# EUROPEAN PATENT SPECIFICATION

(11) **EP 2 437 749 B1**
(45) Date of publication and mention of the grant of the patent: **20.12.2017**
(21) Application number: 10784166.0
(22) Date of filing: 04.06.2010
(51) Int. Cl.: A61K 31/70, A61K 48/00, A61K 38/16, A61P 35/00

(54) **INTERLACED METHOD FOR TREATING CANCER OR A PRECANCEROUS CONDITION**
VERSCHACHTELTES VERFAHREN ZUR BEHANDLUNG VON KREBS ODER EINEM PRÄKANZERÖSEN LEIDEN
PROCÉDÉ COMBINÉ DE TRAITEMENT D'UN CANCER OU D'UN ÉTAT PRÉCANCÉREUX

(30) Priority: 05.06.2009 US 184658 P
(43) Date of publication of application: 11.04.2012
(73) Proprietor: TAU Therapeutics LLC, Charlottesville, Virginia 22902 (US)
(72) Inventor: KROUSE, Andrew J., Charlottesville, Virginia 22911 (US); GRAY, Lloyd S., Louisa, Virginia 23093 (US); MACDONALD, Timothy, Charlottesville, Virginia 22902 (US); LINDEN, Joel, Charlottesville, Virginia 22901 (US)
(74) Representative: Hoffmann Eitle
(86) International application number: PCT/US2010/037437
(87) International publication number: WO 2010/141842

(56) References cited:
- WO-A2-2006/023883
- US-A1- 2006 003 020
- US-A1- 2008 194 669
- MCCALMONT W F; HEADY T N; PATTERSON J R; LINDENMUTH M A; HAVERSTICK D M; GRAY L S; MACDONALD T L: "Design, synthesis, and biological evaluation of novel T-Type calcium channel antagonists", BIOORGANIC AND MEDICINAL CHEMISTRY LETTERS, vol. 14, no. 14, 16 July 2004 (2004-07-16) , pages 3691-3695, XP002692339, DOI: 10.1016/j.bmcl.2004.05.011
- HAVERSTICK D M; HEADY T N; MACDONALD T L; GRAY L S: "Inhibition of human prostate cancer proliferation in vitro and in a mouse model by a compound synthesized to block Ca<2+> entry<1>", CANCER RESEARCH, vol. 60, 15 February 2000 (2000-02-15), pages 1002-1008, XP002692338,
- YOSHIDA, J. ET AL.: 'G1 cell cycle arrest by amlodipine, a dihydropyridine Ca2+ channel blocker, in human epidermoid carcinoma A431 cells.' BIOCHEM. PHARMACOL. vol. 73, no. 7, 14 December 2006, pages 943 - 953, XP005905171
- KANDA, H. ET AL.: 'Naftopidil, a selective alpha-1 adrenoceptor antagonist, inhibits growth of human prostate cancer cells by G1 cell cycle arrest.' INT. J. CANCER. vol. 122, no. 2, 15 January 2008, pages 444 - 451, XP055074821
- ZHANPING, W. ET AL.: 'Voltage-gated K+ channels are associated with cell proliferation and cell cycle of ovarian cancer cell.' GYNECOL. ONCOL. vol. 104, no. 2, 02 October 2006, pages 455 - 460, XP005853890
- KLIMATCHEVA, E. ET AL.: 'Wonderlin, W. F., An ATP-sensitive K(+) current that regulates progression through early G1 phase of the cell cycle in MCF-7 human breast cancer cells.' J. MEMBR. BIOL. vol. 171, no. 1, 01 September 1999, pages 35 - 46, XP055074824
- WANG, Z. ET AL.: 'Ca2+ influx via T-type channels modulates PDGF-induced replication of mouse fibroblasts.' AM. J. PHYSIOL. vol. 265, November 1993, pages C1239 - C1246, XP008164012

## Description

### BACKGROUND OF THE INVENTION

Conventional cancer therapy is rarely curative and can have profound adverse side effects. In part, this stems from the cell cycle specific mechanism of action of most chemotherapeutic drugs, which renders them less effective against a population of cells representing all cell cycle phases.

The cell cycle is the series of events occurring in a cell leading to its division and duplication. In eukaryotic cells, the cycle can be divided into two periods, interphase and mitosis. Transit through these two periods of the cell cycle is known as progression or proliferation. During interphase, the cell grows, accumulates nutrients needed for mitosis and duplicates its DNA. During mitosis, the cell splits itself into two distinct daughter cells. Interphase includes three distinct phases, Gap 1 (G₁) phase, S phase and Gap 2 (G₂) phase while mitosis includes two processes. G₁ phase includes the cell increasing in size, biosynthetic activities of the cell increasing and the synthesis of enzymes needed for DNA replication in the subsequent step. S phase includes the beginning of DNA synthesis and replication of all of the chromosomes. G₂ phase lasts until the cell enters mitosis and includes protein synthesis including the production of microtubules for mitosis. Mitosis includes a process where the cell's chromosomes are divided between the two daughter cells and a cytokinesis process where the original cell's cytoplasm divides forming two distinct daughter cells. The cell cycle also includes a resting phase, typically referred to as G₀. The boundaries between the various phases, for example the boundary between the G₁ and S phase is referred to as a cell cycle checkpoint.

The progression of the cell cycle can be inhibited, so that a particular cell stops the cycle at a point, a cellular checkpoint, before proceeding to the next phase. Cell cycle checkpoints are located between the different phases of the cell cycle, with two of the checkpoints being at the interface between the G₁ and the S phase (G₁/S) and the interface between the G₂ and M phase. A cell cycle inhibitor can stop the progression of a cell from passing to the next phase, for example a cell can be inhibited at the G₁/S cell cycle checkpoint, which forces the cell to remain in the G₁ phase until the inhibitor is removed.

In any particular cancer cell population or tumor in an individual, the length of the cell cycle is variable. This variability is due to differing periods spent in G₁ of G₀ while the length of time from the beginning of S phase to the end of M phase is relatively constant.

Conventional chemotherapeutic treatment only disrupts events in the S or M phase of the cell cycle, leaving cells in the other phases of the cell cycle relatively unharmed. For example, an alkylating agent will act in the S phase while microtubule stabilizing or disruption drugs act in the M phase. Unfortunately, a particular cell is not likely to be at the S or M phase of the cell cycle at a specific time. To compensate for this, chemotherapeutic drugs must be administered repetitively, over long periods of time to increase the chances of reaching a cell which is in the specific cell cycle phase. This repetitive administration translates into larger doses of harmful drugs and an increased toxicity in a subject. US2006/0003020 relates to methods for inhibiting tumor cell metastasis in cancer comprising administering a composition comprising a T-type calcium channel inhibitor, such as mibefradil.

What is desired is a treatment to arrest the cell cycle for a clinically relevant fraction of cells at the CNS cell cycle checkpoint, so that the efficacy of chemotherapeutics can be enhanced.

### SUMMARY OF THE INVENTION

The present invention provides a method for treating a disease or condition in a mammal which comprises the steps of; administering a therapeutically effective amount of a T type calcium channel inhibitor to effectively slow or stop progression of eukaryotic cells through the S, G₂ and M phases of the cell cycle to increase the proportion of the eukaryotic cells in the G₁ phase, stopping administration of the T type calcium channel inhibitor for a period of time, and administering a dosage selected from the group consisting of a dosage of at least one chemotherapeutic agent, a dosage of radiation, and combinations thereof, to kill the proportion of eukaryotic cells progressing past the G₁ phase of the cell cycle after the stopping of the administration of the T type calcium channel inhibitor.

### BRIEF DESCRIPTION OF THE DRAWINGS

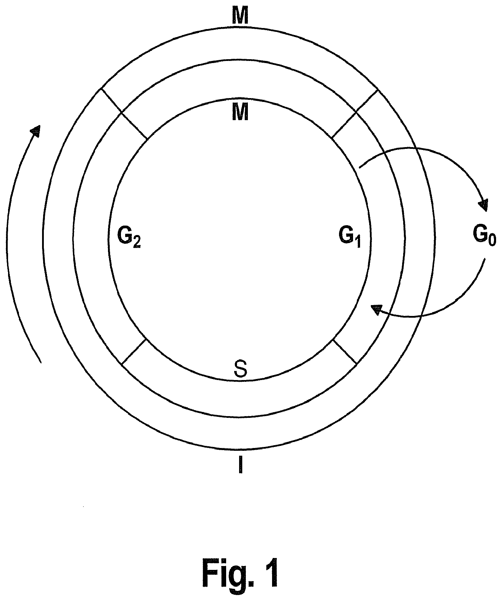

Figure 1 is a schematic representation of the cell cycle. The outer ring includes the interphase (I) and mitosis (M) stages, with the duration of mitosis in relation to the other phases being exaggerated. The inner ring includes the Gap 1 (G₁), Gap 2 (G₂) and synthesis (S) phases. Gap 0 (G₀) or resting phase is not shown.
Figure 2 is a graphical representation of the ability of several chemical agents to inhibit calcium influx as compared to the same chemical agent's ability to inhibit cell proliferation.
Figure 3 is a schematic representation of the cell cycle and the influence several calcium channel blockers have on the progression of the cell cycle.
Figure 4 is a graphical representation of data measured during interlaced therapy.
Figure 5 is a graphical representation of data measured during interlaced therapy.

### DEFINITIONS

In describing and claiming the invention, the following terminology will be used in accordance with the definitions set forth below.

As used herein, the term "treating" includes administering therapy to prevent, cure, ameliorate, reduce, inhibit or alleviate/prevent the symptoms associated with, a specific disorder, disease, injury or condition. For example treating cancer includes inhibition or complete growth arrest of a tumor, reduction in the number of tumor cells, reduction in tumor size, inhibition of tumor cell infiltration into peripheral organs/tissues, inhibition of metastasis as well as relief, to some extent, of one or more symptoms associated with the disorder. The treatment of cancer also includes the administration of a therapeutic agent that directly decreases the pathology of tumor cells, or renders the tumor cells more susceptible to treatment by other therapeutic agents, e.g., radiation and/or chemotherapy. As used herein, the term "treating" includes prophylaxis of the specific disorder or condition, or alleviation of the symptoms associated with a specific disorder or condition and/or preventing or eliminating said symptoms.

As used herein, the term "pharmaceutically acceptable carrier, vehicle or diluent" includes any of the standard pharmaceutical carriers, such as a phosphate buffered saline solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

The term "therapeutically effective amount" means an amount of a compound of the present invention that ameliorates, attenuates, reduces or eliminates a particular disease or condition or prevents or delays the onset of a particular disease or condition.

By "mammal" it is meant to refer to all mammals, including, for example, primates such as humans and monkeys. Examples of other mammals included herein are rabbits, dogs, cats, cattle, goats, sheep, mice, rats and horses. Preferably, the mammal is a female or male human.

The expression "pre-cancerous condition" refers to a growth that is not malignant but is likely to become so if not treated. A "pre-cancerous condition" is also known as "pre-malignant condition" by one of ordinary skill in the art.

It is understood to one skilled in the art that "T type calcium channel blockers" are also known as "T type calcium channel inhibitors".

As used herein, the term "cell cycle inhibitor" refers to a compound which is capable of slowing or stopping progression of a cell or cells in one stage of the cell cycle from progressing to the subsequent stage of the cell cycle.

As used herein, the term "cytotoxin" refers to a compound which is capable of causing necrosis or apoptosis to a cell which is affected by the compound.

### DETAILED DESCRIPTION OF THE INVENTION

Varying lengths of the cell cycle are determined predominately by the time spent in the G₁ phase. Because of this, any particular cell in a population will reside in G₁ for a period of time before the cell enters the S phase of the cell cycle. To stop the cell cycle from continuing past a cell cycle checkpoint, a cell cycle inhibitor, including a T type calcium channel inhibitor can be administered.

The cell cycle inhibitor is first administered to a mammal in a therapeutically effective amount to effectively slow or stop progression of eukaryotic cells through the S, G₂ and M phases of the cell cycle, thereby increasing the proportion of the eukaryotic cells at the cell cycle checkpoint between the G₁ and S phase (G₁/S). The mammal may be a human. This method may be used to treat cancer and pre-cancerous conditions, and cancerous and pre-cancerous tumors in a mammal.

The administration of the cell cycle inhibitor causes asynchronously progressing or proliferating cancer cells in a population to accumulate at G₁/S as they proceed through the cell cycle because their ability to proceed to the S phase is arrested by the cell cycle inhibitor. For a cell to move from G₁ phase to S phase through the cell cycle checkpoint, the cell requires influx of extracellular calcium to trigger biochemical cascades that are necessary for the progression. Removal of calcium from the extracellular medium blocks cell cycle transit for each cell. This blocking can be accomplished through administration of a T type calcium channel inhibitor. Suitable T type calcium channel inhibitors include mibefradil, efonidipine, ethosuxamide, sutinib, TTL-1177 (a proprietary compound, described in reference: Gray, L.S., Perez-Reyes, E., Gomora, J.C., Haverstick, D.M., Shattock, M., McLatchie, L., Harper, J., Brooks, G., Heady, T., and MacDonald, T.L. (2004) Cell Calcium 36, 489-497) and nickel, among others. Thus, each cell persists in G₁ phase as long as it would in the presence of extracellular calcium, but becomes locked in place when G₁/S is reached without calcium, thereby synchronizing cells at G₁/S. Calcium influx to a cell is necessary for progression and transit through the cell cycle. This is further described in Example 1 below.

The administration of the cell cycle inhibitor increases the percentage of cells at G₁/S. Subsequent to this administration, a dosage of at least one chemotherapeutic agent, a dosage of radiation, or a dosage of both are administered, the dosage being targeted to kill cells in the S phase of the cell cycle. The chemotherapeutic agent can be a cancer chemotherapeutic, a cytotoxin or combinations thereof. The cytotoxin can be an alkylating agent. The cancer chemotherapeutic can be an anti-metabolite or an anti-mitotic and can be selected from the following examples; temozolamide (Temo), 5-fluorouracil, 6-mecaptopurine, bleomycin, carboplatin, cisplatin, dacarbazine, doxorubicin, epirubicin, etoposide, hydroxyurea, ifosfamide, irinotecan, topotecan, metotrexate, mitoxantrone, oxaliplatin, paclitaxel, doocetaxol, vinblastine, vincristine, vinorelbine, vindesine, mitomycin C and combinations thereof. The dosage of at least one chemotherapeutic agent can be administered before, after or during a dosage of radiation. The dosage of radiation can be administered before, after or during a dosage of at least one chemotherapeutic. The period between the first administration of the cell cycle inhibitor and the cytotoxin, allows the accumulation of cells at G₁/S of the cell cycle. This method increases the percentage of the cells which are in the S or M phase, thereby increasing the effectiveness of the dosage of at least one chemotherapeutic agent, the dosage of radiation, or the dosage of both and subsequently reducing the toxic load required to kill a predetermined amount of eukaryotic cells.

The cell cycle inhibitor can be administered a second time, after the administration of the dosage of at least one chemotherapeutic agent, the dosage of radiation, or the dosage of both to slow re-growth of targeted cells or tumors so that further administrations of the dosage can be provided as needed. This second administration of a cell cycle inhibitor will resynchronize a percentage of the population of cells at G₁/S.

The cell cycle inhibitor can be administered through several routes including parenteral, intravenous, intramuscular, intraperitoneal, intrathecal, suppository, transdermal, topical, or oral. Oral administration of the cell cycle inhibitor is most preferred. An oral administration can be administered as a dosage unit, typically a pill or capsule along with a pharmaceutically acceptable carrier.

The T type calcium channel inhibitors restrict the influx of extracellular calcium into the cell which is critical for a number of vital cellular processes. The calcium necessary for these processes comes from the extracellular milieu via influx through calcium channels. Calcium channels are grouped into several families based upon sequence analysis, biophysical characteristics and pharmacological sensitivity. These calcium channels have been implicated in regulation of blood pressure, cardiac rhythm and cellular proliferation. Studies also suggest that T-type calcium channels may play an important role in age related macular degeneration. At least one pharmacological agent, mibefradil, has been proven to be clinically effective because of inhibition of T channel function. Inhibitors of calcium entry are useful for treating hypertension, cardiac arrhythmia and clinically deleterious cellular proliferation.

T type calcium channels are present in cells, cell lines and specifically cancer cell lines. Specifically, the Cav3.2 isoform of T type calcium channels has been shown to be aberrantly expressed in breast cancer tissue as compared to normal adjacent breast tissue in Japanese women, as discussed in reference Asaga, S., Ueda, M., Jinno, H., Kikuchi, K., Itano, O., Ikeda, T., and Kitajima, M. (2006) Anticancer Res 26, 35-42.

Cell cycle inhibitors effectively stop or slow progression of eukaryotic cells at cell cycle checkpoints, including G₁/S, which is further explained in Example 2 below. Further, administration of cell cycle inhibitors effectively slows growth or proliferation of a disease or condition, as explained in Example 3 below.

Subsequent to administration of the cell cycle inhibitor, there is a period during which no cell cycle inhibitor is added. This period can range from about 0 hours to about 336 hours. This period allows the cells which have accumulated at G₁/S to enter the S phase of the cell cycle. Through administration of the cell cycle inhibitor, about 5% to about 25% of cells will have accumulated at G₁/S. The increase in number of cells in the S phase makes an administered dosage of at least one chemotherapeutic agent, dosage of radiation, or the dosage of both more effective because a large percentage of cells will be affected by each dose. By "affected" is meant killed or distilled.

Subsequent to the period where no cell cycle inhibitor is added, a dosage of at least one chemotherapeutic agent, a dosage of radiation, or a dosage of both is administered to kill a proportion of cells in the S. By "killed" is meant that the cell undergoes apoptosis or necrosis. The specific dosage of at least one chemotherapeutic agent, dosage of radiation, or dosage of both will be dictated by clinical experience of one skilled in the art, where different diseases are treated with different dosages and different agents. For exemplary purposes only, the following dosages of chemotherapeutic agents may be used to treat the following diseases. In treating glioblastoma, the cytotoxin temozolamide may be used. In treating melanoma, the cytotoxin melphalan or temozolamide may be used. In treating pancreatic cancer, the cytotoxin gemcitabine may be used. In treating breast cancer, the cytotoxin gemcitabine may be used. In treating colon cancer, the cytotoxin irinotecan or 5-fluorouracil may be used.

This interlaced therapy, i.e. administration of cell cycle inhibitors followed by administration of a dosage of at least one chemotherapeutic agent, a dosage of radiation, or a dosage of both can be used to reduce the progression, proliferation or growth of a disease or condition, as explained more thoroughly in Examples 4 and 5 below. In using this interlaced therapy, the same dosage of at least one chemotherapeutic agent, dosage of radiation, or dosage of both is more effective as compared to a chemotherapeutic or radiation dosage when used alone.

### Example 1

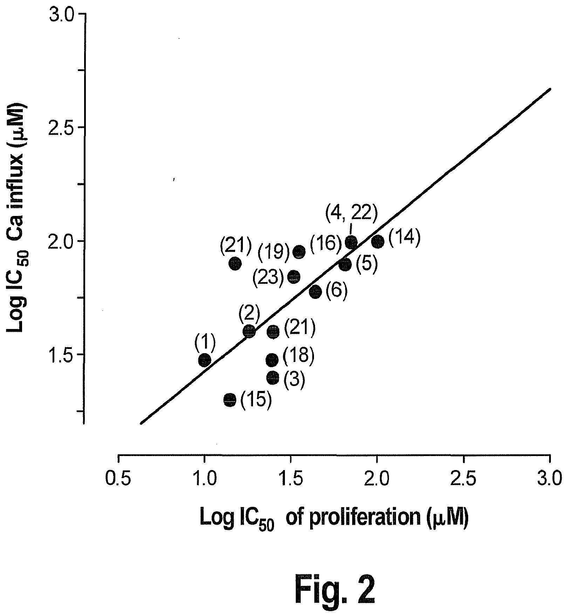

To measure the correspondence between calcium influx inhibition and progression or proliferation, the ability of several chemical agents to inhibit calcium influx was plotted against the ability of the same agent to inhibit proliferation, as can be seen in FIG. 2 below. These agents were proprietary chemical entities synthesized at the University of Virginia. A least squares correlation line with a slope of 0.98 and R² value of 0.92 was obtained. Conventionally, a correlation cannot be used to infer causality, but in this case, a Bayesian approach is warranted because calcium influx is necessary *a priori* for proliferation, such that blocking calcium entry will correspondingly block proliferation. A regression line with a slope very near 1 means that essentially all of the variation in the variables is accounted for by variation in the other, meaning that there is no action of these agents on proliferation other than inhibition of calcium entry.

### Example 2

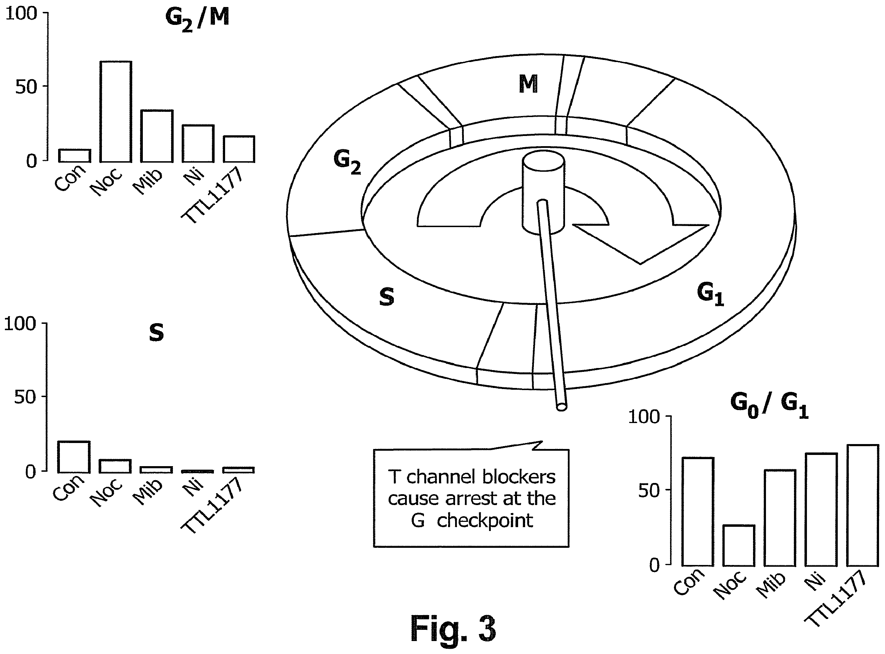

Cell cycle analysis was performed using flow cytometry and BUdR staining. In FIG. 3, "Con" represents untreated, control cells. All other cell cultures were treated with nocodazole, which interferes with microtubule polymerization and blocks the cell cycle during M phase. Treatment of A10 cells with nocodazole alone blocked cell cycle transit through M phase as determined by flow cytometry. Because A10 cells reside primarily in the cell cycle checkpoint between G₀ and G₁ (See "Con" in FIG. 3), evaluating the possibility that a pharmacologic agent inhibits transit out of that phase is not as straightforward as determining blockade at other cell cycle checkpoints. Therefore, cell cultures were treated for 24 hours with T channel calcium channel blockers mibefradil (mib), nickel (Ni) or TTL-1177 (a proprietary compound owned by the assignee Tau Therapeutics), before adding nocodazole. Absent an effect on the T channel blockers, cells would be locked at the cell cycle checkpoint between G₂ and M by nocodazole as happened with cells treated with it alone. Instead, treatment with the T type calcium channel blockers arrested cells at the cell cycle checkpoint between G₀ and G₁ and prevented accumulation at the cell cycle checkpoint between G₂ and M that would otherwise have resulted from nocodazole. This shows that T type calcium channel blockers arrest cycling cells at G₁/S.

### Example 3

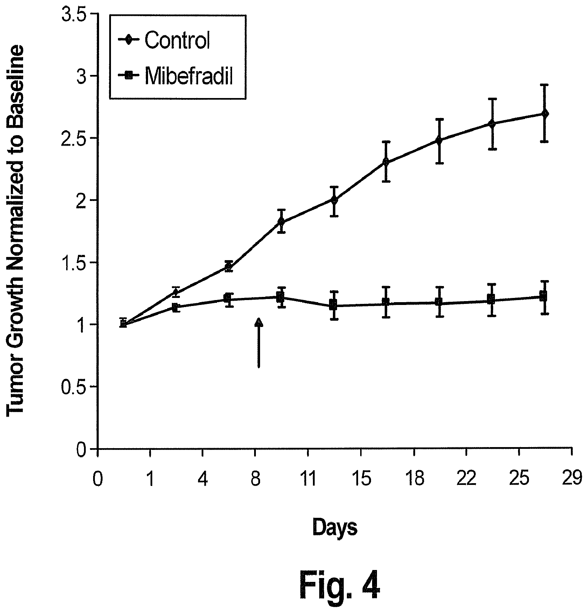

Pancreatic cancers resected from patients were immediately transplanted into the flanks of nude mice and maintained by serial, *in vivo* passage. Transplanted tumors were implanted at a volume of about 100 mm³ and allowed to grow to 200 to 300 mm³ before initiation of treatment. Mice bearing the PANC 219 tumor were either left untreated or treated with mibefradil at 65 mg/kg p.o. b.i.d. (n=10 in each group). Tumor growth was normalized to the size measured at the start of treatment. These results are shown in FIG. 4. One mouse in the treatment group died on day 9 (D9) as indicated by the arrow in FIG. 4. Distinct from conventional chemotherapeutic drugs, treatment with T type calcium inhibitors did not cause tumor regression. Treatment, instead, controlled tumor growth, by arrest of tumor cells at G₁/S.

### Example 4

Studies of interlaced therapy for human glioblastoma in a murine xenograft model using the D54 cell line were conducted. Mice bearing subcutaneous implants of D54 tumors, which were allowed to become established at a volume of about 100 mm³, were treated with mibefradil at 40 mg/kg p.o. q.i.d. for 7 days or left untreated. On day seven, all animals received the S phase cytotoxin temozolamide at a dose of 25% of the LD₁₀ over five days. Following two days of no treatment, mibefradil was re-started at a dose of 35 mg/kg p.o. q.i.d. in the group of mice which were originally treated with mibefradil. On day 20, the mean tumor volume in the temozolamide only group of mice was 173 mm³ as compared to the temozolamide plus mibefradil group of mice, whose mean tumor volume was 102 mm³ (p=0.0485; n=10/group; Student's pooled, two tailed t-test). A pooled, two tailed Student's t-test over all seven tumor volume measurements yielded p=0.0329. There were no animals that were euthanized in either group due to tumor size. The mean volume in the control group, which received no intervention, was 1214 mm³ with three animals euthanized because of tumor size.

### Example 5

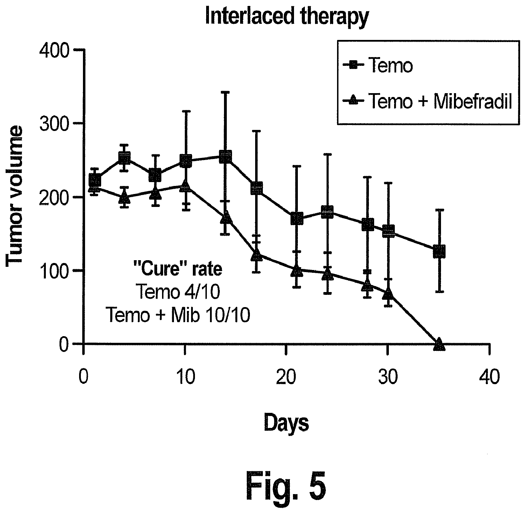

Further studies of interlaced therapy for human glioblastoma in a murine xenograft model using the D54 cell line were conducted. Mice bearing subcutaneous implants of D54 tumors, which were allowed to become established, were treated with mibefradil at 40 mg/kg p.o. q.i.d. for 7 days or left untreated. On day seven, all animals received the S phase cytotoxin temozolamide at a dose of 25% of the LD₁₀ over five days. Following two days of no treatment, mibefradil was re-started at a dose of 35 mg/kg p.o. q.i.d. in the group of mice which were originally treated with mibefradil. Following five days of mibefradil treatment, temozolamide was again administered for 5 days, but at a dose that is 10% of LD₁₀, because the cytotoxin had made the tumors in six of twenty mice too small to measure following the first round of cytotoxin treatment. The results are shown below in FIG. 5. The mice treated with temozolamide alone had tumors with a mean volume of 156 ±65 mm³ vs. a volume of 70 ± 19 mm³ for animals treated with mibefradil and temozolamide. The differences in volume indicate that mice treated with interlacing therapy have tumors about 55% smaller than mice treated with only a cytotoxin over 30 days. By day 35, all the mice in the interlaced group had cancers that could not be palpated. For a two tailed t test at both days 30 and 35, p<0.0001. The non-parametric Wilcoxon signed rank test gave the same p value of 0.00010 at day 35. In part, this reflects the fact that all of the tumors in the interlaced group have regressed to an unpalatable size.

## Claims

1. A T-type calcium channel inhibitor for use to increase the efficacy of killing of proliferating cells by a cancer chemotherapeutic agent or radiation in the treatment of a cancerous or pre-cancerous disease or condition in a mammal, wherein the treatment comprises:
(a) administering a therapeutically effective amount of a T type calcium channel inhibitor to increase the proportion of the proliferating cells in the G₁ phase;
(b) stopping administration of the T type calcium channel inhibitor for a period of time; and
(c) subsequently administering a dosage selected from the group consisting of a dosage of at least one cancer chemotherapeutic agent, a dosage of radiation, or a combination of at least one cancer chemotherapeutic agent and a dosage of radiation and combinations thereof, to kill the proportion of cells progressing past the G₁ phase of the cell cycle after the stopping of the administration of the T type calcium channel inhibitor; and
(d) optionally administering a therapeutically effective amount of a T type calcium channel inhibitor after administering the dosage selected from the group consisting of a dosage of at least one chemotherapeutic agent, a dosage of radiation, and combinations thereof.

2. The T-type calcium channel inhibitor of claim 1 for the use according to claim 1, wherein the treatment comprises (d) administering a therapeutically effective amount of a T type calcium channel inhibitor after administering the dosage selected from the group consisting of a dosage of at least one chemotherapeutic agent, a dosage of radiation, and combinations thereof.

3. The T-type calcium channel inhibitor of claim 1 for the use according to claim 1, wherein the treatment is an extended treatment wherein steps (a) -(c) are repeated one or more times.

4. The T-type calcium channel inhibitor of any one of claims 1 to 3 for the use according to claim 1 to 3, wherein the cancer or pre-cancerous disease or condition is a tumor.

5. The T-type calcium channel inhibitor of any one of claims 1 to 4 for the use according to any one of claims 1 to 4, wherein the period of time between stopping treatment with the T-type calcium channel inhibitor and subsequently administering to the mammal the dosage of at least one cancer chemotherapeutic agent, the dosage of radiation or the combination of at least one cancer chemotherapeutic agent and radiation is about 0 hours to about 336 hours.

6. The T-type calcium channel inhibitor of any one of claims 1 to 5 for the use according to any one of claims 1 to 5, wherein the cancerous or pre-cancerous disease or condition is selected from the group consisting of glioblastoma, melanoma, pancreatic cancer, breast cancer and colon cancer.

7. The T-type calcium channel inhibitor of any one of claims 1 to 6 for the use according to any one of claims 1 to 6, wherein the T type calcium channel inhibitor is selected from mibefradil, efonidipine, ethosuximide, sunitinib, TTL-1177 and nickel.

8. The T-type calcium channel inhibitor of claim 7 for the use according to claim 7, wherein the T type calcium channel inhibitor is mibefradil.

9. The T-type calcium channel inhibitor of any one of claims 1 to 8 for the use according to any one of claims 1 to 8, wherein the treatment comprises administering a dosage of at least one chemotherapeutic agent in step (b).

10. The T-type calcium channel inhibitor of any one of claims 1 to 9 for the use according to any one of claims 1 to 9, wherein the treatment comprises treatment with a cancer chemotherapeutic agent selected from the group consisting of temozolomide, 5-fluorouracil, 6-mercaptopurine, bleomycin, carboplatin, cisplatin, dacarbazine, doxorubicin, epirubicin, etoposide, hydroxyurea, ifosfamide, irinotecan, topotecan, methotrexate, mitoxantrone, oxaliplatin, paclitaxel, docetaxel, vinblastine, vincristine, vinorelbine, vindesine, mitomycin C and combinations thereof.

11. The T-type calcium channel inhibitor of any one of claims 1 to 8 for the use according to any one of claims 1 to 8, wherein the treatment comprises administering a dosage of radiation in step (b).

12. The T-type calcium channel inhibitor of any one of claims 1 to 10 for the use according to any one of claims 1 to 10, wherein the at least one chemotherapeutic agent is temozolomide.

13. The T-type calcium channel inhibitor of claim 12 for the use according to claim 12, wherein the cancer or pre-cancerous disease or condition is glioblastoma.

14. The T-type calcium channel inhibitor of any one of claims 1 to 10 for the use according to any one of claims 1 to 10, wherein the at least one chemotherapeutic agent is melphalan.

15. The T-type calcium channel inhibitor of claim 12 or 14 for the use according to claim 12 or 14, wherein the cancer or pre-cancerous disease or condition is melanoma.

16. The T-type calcium channel inhibitor of any one of claims 1 to 10 for the use according to any one of claims 1 to 10, wherein the at least one chemotherapeutic agent is carboplatin.

17. The T-type calcium channel inhibitor of any one of claims 1 to 10 for the use according to any one of claims 1 to 10, wherein the at least one chemotherapeutic agent is gemcitabine.

18. The T-type calcium channel inhibitor of claim 17 for the use according to claim 17, wherein the cancer or pre-cancerous disease or condition is pancreatic cancer.

19. The T-type calcium channel inhibitor of claim 17 for the use according to claim 17, wherein the cancer or pre-cancerous disease or condition is breast cancer.

20. The T-type calcium channel inhibitor of any one of claims 1 to 10 for the use according to any one of claims 1 to 10, wherein the at least one chemotherapeutic agent is irinotecan.

21. The T-type calcium channel inhibitor of any one of claims 1 to 10 for the use according to any one of claims 1 to 10, wherein the at least one chemotherapeutic agent is 5-fluorouracil.

22. The T-type calcium channel inhibitor of claim 20 or 21 for the use according to claim 20 or 21, wherein the cancer or pre-cancerous disease or condition is colon cancer.

23. The T-type calcium channel inhibitor of any one of claims 1 to 22 for the use according to any one of claims 1 to 22, wherein the mammal is a human.

## Patentansprüche

1. T-Typ-Calciumkanalinhibitor zur Verwendung, um die Wirksamkeit des Abtötens von proliferierenden Zellen durch ein krebs-chemotherapeutisches Agens oder Strahlung bei der Behandlung einer/eines kanzerösen oder präkanzerösen Erkrankung oder Zustands in einem Säugetier zu erhöhen, wobei die Behandlung umfasst:
(a) das Verabreichen einer therapeutisch wirksamen Menge eines T-Typ-Calciumkanalinhibitors, um den Anteil der proliferierenden Zellen in der G₁-Phase zu erhöhen;
(b) das Beenden der Verabreichung des T-Typ-Calciumkanalinhibitors für eine Zeitspanne; und
(c) nachfolgend das Verabreichen einer Dosierung, die ausgewählt ist aus der Gruppe bestehend aus einer Dosierung von mindestens einem krebs-chemotherapeutischen Agens, einer Strahlungsdosierung, oder einer Kombination von mindestens einem krebs-chemotherapeutischen Agens und einer Strahlungsdosierung und Kombinationen davon, um den Anteil an Zellen abzutöten, die über die G₁-Phase des Zellzyklus hinaus fortschreiten, nach dem Beenden der Verabreichung des T-Typ-Calciumkanalinhibitors; und
(d) optional das Verabreichen einer therapeutisch wirksamen Menge eines T-Typ-Calciumkanalinhibitors nach dem Verabreichen der Dosierung, die ausgewählt ist aus der Gruppe bestehend aus einer Dosierung von mindestens einem chemotherapeutischen Agens, einer Strahlungsdosierung und Kombinationen davon.

2. T-Typ-Calciumkanalinhibitor gemäß Anspruch 1 zur Verwendung gemäß Anspruch 1, wobei die Behandlung (d) das Verabreichen einer therapeutisch wirksamen Menge eines T-Typ-Calciumkanalinhibitors nach dem Verabreichen der Dosierung, die ausgewählt ist aus der Gruppe bestehend aus einer Dosierung von mindestens einem chemotherapeutischen Agens, einer Strahlungsdosierung und Kombinationen davon, umfasst.

3. T-Typ-Calciumkanalinhibitor gemäß Anspruch 1 zur Verwendung gemäß Anspruch 1, wobei die Behandlung eine erweiterte Behandlung ist, wobei Schritte (a)-(c) einmal oder mehrmals wiederholt werden.

4. T-Typ-Calciumkanalinhibitor gemäß irgendeinem der Ansprüche 1 bis 3 zur Verwendung gemäß der Ansprüche 1 bis 3, wobei der Krebs oder die/der präkanzeröse Erkrankung oder Zustand ein Tumor ist.

5. T-Typ-Calciumkanalinhibitor gemäß irgendeinem der Ansprüche 1 bis 4 zur Verwendung gemäß irgendeinem der Ansprüche 1 bis 4, wobei die Zeitspanne zwischen dem Beenden der Behandlung mit dem T-Typ-Calciumkanalinhibitor und dem nachfolgenden Verabreichen der Dosierung von mindestens einem krebs-chemotherapeutischen Agens, der Strahlungsdosierung oder der Kombination von mindestens einem krebs-chemotherapeutischen Agens und Strahlung an das Säugetier etwa 0 Stunden bis etwa 336 Stunden beträgt.

6. T-Typ-Calciumkanalinhibitor gemäß irgendeinem der Ansprüche 1 bis 5 zur Verwendung gemäß irgendeinem der Ansprüche 1 bis 5, wobei die/der kanzeröse oder präkanzeröse Erkrankung oder Zustand ausgewählt ist aus der Gruppe bestehend aus Glioblastom, Melanom, Bauchspeicheldrüsenkrebs, Brustkrebs und Dickdarmkrebs.

7. T-Typ-Calciumkanalinhibitor gemäß irgendeinem der Ansprüche 1 bis 6 zur Verwendung gemäß irgendeinem der Ansprüche 1 bis 6, wobei der T-Typ-Calciumkanalinhibitor ausgewählt ist aus Mibefradil, Efonidipin, Ethosuximid, Sunitinib, TTL-1177 und Nickel.

8. T-Typ-Calciumkanalinhibitor gemäß Anspruch 7 zur Verwendung gemäß Anspruch 7, wobei der T-Typ-Calciumkanalinhibitor Mibefradil ist.

9. T-Typ-Calciumkanalinhibitor gemäß irgendeinem der Ansprüche 1 bis 8 zur Verwendung gemäß irgendeinem der Ansprüche 1 bis 8, wobei die Behandlung das Verabreichen einer Dosierung von mindestens einem chemotherapeutischen Agens in Schritt (b) umfasst.

10. T-Typ-Calciumkanalinhibitor gemäß irgendeinem der Ansprüche 1 bis 9 zur Verwendung gemäß irgendeinem der Ansprüche 1 bis 9, wobei die Behandlung eine Behandlung mit einem krebs-chemotherapeutischen Agens umfasst, das ausgewählt ist aus der Gruppe bestehend aus Temozolomid, 5-Fluorouracil, 6-Mercaptopurin, Bleomycin, Carboplatin, Cisplatin, Dacarbazin, Doxorubicin, Epirubicin, Etoposid, Hydroxyurea, Ifosfamid, Irinotecan, Topotecan, Methotrexat, Mitoxantron, Oxaliplatin, Paclitaxel, Docetaxel, Vinblastin, Vincristin, Vinorelbin, Vindesin, Mitomycin C und Kombinationen davon.

11. T-Typ-Calciumkanalinhibitor gemäß irgendeinem der Ansprüche 1 bis 8 zur Verwendung gemäß irgendeinem der Ansprüche 1 bis 8, wobei die Behandlung das Verabreichen einer Strahlungsdosierung in Schritt (b) umfasst.

12. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens Temozolomid ist.

13. T-Typ-Calciumkanalinhibitor gemäß Anspruch 12 zur Verwendung gemäß Anspruch 12, wobei der Krebs oder die/der präkanzeröse Erkrankung oder Zustand Glioblastom ist.

14. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens Melphalan ist.

15. T-Typ-Calciumkanalinhibitor gemäß Anspruch 12 oder 14 zur Verwendung gemäß Anspruch 12 oder 14, wobei der Krebs oder die/der präkanzeröse Erkrankung oder Zustand Melanom ist.

16. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens Carboplatin ist.

17. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens Gemcitabin ist.

18. T-Typ-Calciumkanalinhibitor gemäß Anspruch 17 zur Verwendung gemäß Anspruch 17, wobei der Krebs oder die/der präkanzeröse Erkrankung oder Zustand Bauchspeicheldrüsenkrebs ist.

19. T-Typ-Calciumkanalinhibitor gemäß Anspruch 17 zur Verwendung gemäß Anspruch 17, wobei der Krebs oder die/der präkanzeröse Erkrankung oder Zustand Brustkrebs ist.

20. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens Irinotecan ist.

21. T-Typ-Calciumkanalinhibitor gemäß irgendeinem der Ansprüche 1 bis 10 zur Verwendung gemäß irgendeinem der Ansprüche 1 bis 10, wobei das mindestens eine chemotherapeutische Agens 5-Fluorouracil ist.

22. T-Typ-Calciumkanalinhibitor gemäß Anspruch 20 oder 21 zur Verwendung gemäß Anspruch 20 oder 21, wobei der Krebs oder die/der präkanzeröse Erkrankung oder Zustand Dickdarmkrebs ist.

23. T-Typ-Calciumkanalinhibitor gemäß irgendeinem der Ansprüche 1 bis 22 zur Verwendung gemäß irgendeinem der Ansprüche 1 bis 22, wobei das Säugetier ein Mensch ist.

## Revendications

1. Inhibiteur des canaux calciques de type T pour l'utilisation afin d'augmenter l'efficacité de destruction de cellules prolifératives par un agent chimio thérapeutique anticancéreux ou des radiations dans le traitement d'une maladie ou affection cancéreuse ou précancéreuse dans un mammifère, dans laquelle le traitement comprend:
(a) l'administration d'une quantité thérapeutiquement efficace d'un inhibiteur des canaux calciques de type T pour augmenter la proportion des cellules prolifératives dans la phase G₁ ;
(b) l'arrêt de l'administration de l'inhibiteur des canaux calciques de type T pendant une période de temps ; et
(c) l'administration ultérieure d'une dose choisie parmi le groupe constitué d'une dose d'au moins un agent chimio thérapeutique anticancéreux, une dose de radiations, ou une combinaison d'au moins un agent chimio thérapeutique anticancéreux et une dose de radiations et des combinaisons de ceux-ci, afin de tuer la proportion de cellules progressant après la phase G₁ du cycle cellulaire après l'arrêt de l'administration de l'inhibiteur des canaux calciques ; et
(d) optionnellement l'administration d'une quantité thérapeutiquement efficace d'un inhibiteur des canaux calciques de type T après l'administration de la dose choisie parmi le groupe constitué d'une dose d'au moins un agent chimio thérapeutique, une dose de radiations, et des combinaisons de ceux-ci.

2. L'inhibiteur des canaux calciques de type T de la revendication 1 pour l'utilisation selon la revendication 1, dans laquelle le traitement comprend (d) l'administration d'une quantité thérapeutiquement efficace d'un inhibiteur des canaux calciques de type T après l'administration de la dose choisie parmi le groupe constitué d'une dose d'au moins un agent chimio thérapeutique, une dose de radiations, et des combinaison de ceux-ci.

3. L'inhibiteur des canaux calciques de type T de la revendication 1 pour l'utilisation selon la revendication 1, dans laquelle le traitement est un traitement étendu dans lequel les étapes (a)-(c) sont répétées une ou plusieurs fois.

4. L'inhibiteur des canaux calciques de type T de l'une quelconque des revendications 1 à 3 pour l'utilisation selon les revendications 1 à 3, dans lequel la maladie ou affection cancéreuse ou précancéreuse est une tumeur.

5. L'inhibiteur des canaux calciques de type T de l'une quelconque des revendications 1 à 4 pour l'utilisation selon l'une quelconque des revendications 1 à 4, dans laquelle la période de temps entre l'arrêt du traitement avec l'inhibiteur des canaux calciques de type T et l'administration ultérieure au mammifère de la dose d'au moins un agent chimio thérapeutique anticancéreux, la dose de radiations ou la combinaison d'au moins un agent chimio thérapeutique anticancéreux et de radiations est d'environ 0 heure à environ 336 heures.

6. L'inhibiteur des canaux calciques de type T de l'une quelconque des revendications 1 à 5 pour l'utilisation selon les revendications 1 à 5, dans laquelle la maladie ou affection cancéreuse ou précancéreuse est choisie parmi le groupe constitué du glioblastome, du mélanome, du cancer pancréatique, du cancer du sein et du cancer du côlon.

7. L'inhibiteur des canaux calciques de type T de l'une quelconque des revendications 1 à 6 pour l'utilisation selon les revendications 1 à 6, dans laquelle l'inhibiteur des canaux calciques de type T est choisi parmi le mibéfradil, l'éfonidipine, l'éthosuximide, le sunitinib, le TTL-1177 et le nickel.

8. L'inhibiteur des canaux calciques de type T selon la revendication 7 pour l'utilisation selon la revendication 7, dans laquelle l'inhibiteur des canaux calciques de type T est le mibéfradil.

9. L'inhibiteur des canaux calciques de type T de l'une quelconque des revendications 1 à 8 pour l'utilisation selon les revendications 1 à 8, dans laquelle le traitement comprend l'administration d'une dose d'au moins un agent chimio thérapeutique dans l'étape (b).

10. L'inhibiteur des canaux calciques de type T de l'une quelconque des revendications 1 à 9 pour l'utilisation selon les revendications 1 à 9, dans laquelle le traitement comprend un traitement avec un agent chimio thérapeutique anticancéreux choisi parmi le groupe constitué du témozolomide, du 5-fluorouracil, de la 6-mercaptopurine, de la bléomycine, du carboplatine, du cisplatine, de la dacarbazine, de la doxorubicine, de l'épirubicine, de l'étoposide, de l'hydroxyurée, de l'ifosfamide, de l'irinotécan, du topotécan, du méthotrexate, de la mitoxantrone, de l'oxaliplatine, du paclitaxel, du docétaxel, de la vinblastine, de la vincristine, de la vinorelbine, de la vindésine, de la mitomycine C et des combinaisons de ceux-ci.

11. L'inhibiteur des canaux calciques de type T de l'une quelconque des revendications 1 à 8 pour l'utilisation selon les revendications 1 à 8, dans laquelle le traitement comprend l'administration d'une dose de radiations dans l'étape (b).

12. L'inhibiteur des canaux calciques de type T de l'une quelconque des revendications 1 à 10 pour l'utilisation selon les revendications 1 à 10, dans laquelle le au moins un agent chimio thérapeutique est le témozolomide.

13. L'inhibiteur des canaux calciques de type T de la revendication 12 pour l'utilisation selon la revendication 12, dans laquelle la maladie ou affection cancéreuse ou précancéreuse est le glioblastome.

14. L'inhibiteur des canaux calciques de type T de l'une quelconque des revendications 1 à 10 pour l'utilisation selon les revendications 1 à 10, dans laquelle le au moins un agent chimio thérapeutique est le melphalan.

15. L'inhibiteur des canaux calciques de type T de la revendication 12 ou 14 pour l'utilisation selon la revendication 12 ou 14, dans laquelle la maladie ou affection cancéreuse ou précancéreuse est le mélanome.

16. L'inhibiteur des canaux calciques de type T de l'une quelconque des revendications 1 à 10 pour l'utilisation selon les revendications 1 à 10, dans laquelle le au moins un agent chimio thérapeutique est le carboplatine.

17. L'inhibiteur des canaux calciques de type T de l'une quelconque des revendications 1 à 10 pour l'utilisation selon les revendications 1 à 10, dans laquelle le au moins un agent chimio thérapeutique est la gemcitabine.

18. L'inhibiteur des canaux calciques de type T de la revendication 17 pour l'utilisation selon la revendication 17, dans laquelle la maladie ou affection cancéreuse ou précancéreuse est le cancer pancréatique.

19. L'inhibiteur des canaux calciques de type T de la revendication 17 pour l'utilisation selon la revendication 17, dans laquelle la maladie ou affection cancéreuse ou précancéreuse est le cancer du sein.

20. L'inhibiteur des canaux calciques de type T de l'une quelconque des revendications 1 à 10 pour l'utilisation selon les revendications 1 à 10, dans laquelle le au moins un agent chimio thérapeutique est l'irinotécan.

21. L'inhibiteur des canaux calciques de type T de l'une quelconque des revendications 1 à 10 pour l'utilisation selon les revendications 1 à 10, dans laquelle le au moins un agent chimio thérapeutique est le 5-fluorouracil.

22. L'inhibiteur des canaux calciques de type T de la revendication 20 ou 21 pour l'utilisation selon la revendication 20 ou 21, dans laquelle la maladie ou affection cancéreuse ou précancéreuse est le cancer du côlon.

23. L'inhibiteur des canaux calciques de type T de l'une quelconque des revendications 1 à 22 pour l'utilisation selon l'une quelconque des revendications 1 à 22, dans laquelle le mammifère est un humain.
